# EUROPÄISCHE PATENTANMELDUNG

(11) **EP 1 224 941 A2**
(43) Veröffentlichungstag der Anmeldung: **24.07.2002**
(21) Anmeldenummer: 01130770.9
(22) Anmeldetag: 22.12.2001
(51) Int. Cl.: A61K 38/57, A61P 25/02, A61P 31/08, A61P 31/12, A61P 35/00, A61P 13/12, A61P 19/02

(54) **Antithrombin III gegen durch Angiogenese verursachte Erkrankungen**

(30) Priorität: 17.01.2001 DE 10102048
(71) Anmelder: Aventis Behring GmbH, 35002 Marburg (DE)
(72) Erfinder: Robinson, Jane C., Datchworth, Hertfordshire SG3 6RR (GB); Gray, Elaine, Hanwell, London W7 1PB (GB); Wiedermann, Christian Josef, 6020 Innsbruck (AT); Roemisch, Juergen, 35041 Marburg (DE); Stauss, Harald, 35232 Dautphetal (DE)

(57) **Zusammenfassung**

Es wird die Verwendung des aktiven Antithrombin III, das Thrombin inhibitorische Eigenschaften und Affinität zu Heparin hat, für die Prophylaxe und Behandlung von Erkrankungen, die durch eine pathologische Angiogenese oder Arteriogenese erzeugt werden, beschrieben.

## Beschreibung

Gegenstand der Erfindung ist die Verwendung der anti-angiogenen und antiarteriogenen Wirksamkeit von Antithrombin III zur Prophylaxe und Behandlung verschiedener Erkrankungen.

Unter der Angiogenese versteht man das Wachstum von Gefäßkapillaren und das Wachsen endothelialer Kanäle, während die Arteriogenese das Wachstum von bereits vorhandenen Kollateralgefäßen bezeichnet, verbunden mit der Ausdehnung der vorhandenen, mit Muskeln versehenen Arterien (1). Beide Vorgänge werden durch die Bindung von angiogen wirksamen Substanzen an Rezeptoren ausgelöst, die sich auf endothelialen Zellen befinden, welche sich dann vermehren und abwandern. Parallel dazu erhöhen stimulierte endotheliale Zellen auch die Bildung von Adhäsionsmolekülen (Integrine) wie αᵥβ_{δ}, welche zur Verankerung der ausgewanderten endothelialen Zellen am umgebenden Gewebe dienen, wodurch Sprosse von neuen Blutgefäßen gebildet werden. Zusätzlich werden Metalloproteinasen gebildet, die das umgebende Gewebe auflösen und damit die Neubildung des Gewebes um die Blutgefäße herum ermöglichen. Die sprossenden endothelialen Zellen dringen in röhrenförmige und schleifenförmige Vertiefungen ein und ermöglichen so die Bildung von Blutgefäßen. Da angiogene Wirkstoffe eine entscheidende Rolle bei der Angiogenese und Arteriogenese spielen, hat die Verstärkung oder Verminderung ihrer Produktion und Wirkungen einen hohen Einfluss auf die normale physiologische Kontrolle dieser Vorgänge und auf Erkrankungen, die von der Angiogenese beeinflusst werden. Die pathologische Angiogenese ist ein Kennzeichen von Krebs und verschiedenen ischämischen und entzündlichen Erkrankungen. Es gibt Beweise für die wichtige Rolle von angiogen wirksamen Substanzen und Wachstumsfaktoren beim Wachstum und der Bildung von Metastasen von Krebszellen (2). Es ist gesichert, dass eine übermäßige Angiogenese zu Krankheiten wie diabetischer Retinopathie, Neuropathie, rheumatoider Arthritis, Psoriasis und Endometriosis führen kann. Die Angiogenese trägt zu pathophysiologischen Gewebeveränderungen bei der chronischen Bronchitis und chronischen Entzündungen des Magen-Darm-Traktes sowie zu granulomatösen und anderen infektösen Erkrankungen wie Lepra bei.

Antithrombin ist einer der wichtigsten endogenen Inhibitoren der Koagulation. Obwohl Antithrombin vor allem als wichtiger plasmatischer Inhibitor von Thrombin wirkt, hat er auch starke inhibitorische Wirkungen auf eine Anzahl von aktiven Serinproteasen einschließlich der Faktoren IXla, Xa, Xla und Xlla und auf den an den Gewebefaktor gebundenen Faktor Vlla, die alle für die Koagulationskaskäde wichtig sind. Zwei Isoformen des Antithrombins sind in humanem Plasma identifiziert worden. Die β-Isoform macht 5 bis 10% des plasmatischen Antithrombins aus und hat eine höhere Heparinaffinität als die a-Isoform. Allerdings variieren die Anteile dieser beider Isoformen mit dem Gewebe, aus dem sie isoliert werden (3) und in Abhängigkeit von den angewendeten Isolierungsverfahren enthalten unterschiedliche Antithrombinkonzentrate auch unterschiedliche Mengen der Isoformen (4).

Kürzlich beschrieben O'Reilly et al. (5) die anti-angiogene und anti-Tumorwirksamkeit der gespaltenen und der latenten Form von Antithrombin, während das aktive Antithrombin (AT) solche Eigenschaften nicht zeigte. Durch Fraktionierung des Überstandes der Zellkultur wurde von O'Reilly et al. ein neues anti-angiogenes Protein gefunden, das als Antithrombin identifiziert und in dem sog. aktiven 'Loop' gespalten wurde, was zu einem Verlust seiner inhibitorischen Eigenschaften gegenüber den bekannten Proteasen wie Thrombin führte. Dieser proteolytische Schnitt wurde durch Elastase nachvollzogen. Die Äenderung der Konformation von AT nach der Isolierung kann in einer ähnlichen Weise durch Hitzebehandlung herbeigeführt werden und ergibt dann das sog. "locked oder latente" AT.

Überraschenderweise wurde nun gefunden, dass die aktive Form des AT, die durch intakte Moleküle mit der Fähigkeit Proteasen wie Thrombin und den Faktor Xla zu inhibieren und durch eine starke Interaktion mit Heparin und verwandten Verbindungen definiert ist, sowohl anti-angiogene als auch anti-arteriogene Eigenschaften hat. Deshalb kann die aktive Form von AT als Medikament für die Prophylaxe und Behandlung von Erkrankungen eingesetzt werden, die durch eine pathologische Angiogenese und Arteriogenese entstehen.

In einer Serie von Experimenten wurden zunächst die inhibitorischen Wirkungen der aktiven Formen von Antithrombin einschließlich der α- und β-Formen des Antithrombins, auf durch Wachstumsfaktoren induzierte endotheliale Zellvermehrung untersucht. Dann wurden die Wirkungen dieser aktiven Isoformen auf die durch Serum induzierte Vermehrung der endothelialen Zellen der menschlichen Nabelschnurvene (HUVEC) und der arteriellen Zellen der Kalbslunge (CPAC) untersucht. AT α und β wurden durch fraktionierte Chromatographie unter Verwendung einer Heparinmatrix hergestellt. Unter diesen Bedingungen erschien das latente Antithrombin in der die Säule durchfließenden Fraktion, während die α-lsoform durch Elution mit 0,8 M NaCI und die β-lsoform anschließend durch Elution mit 2 M NaCI erhalten wurde. Unter Anwendung der sog. zweidimensionalen Immun-Elektrophorese (in Gegenwart von Heparin) wurde die Abwesenheit des latenten/'locked' AT in den beiden letztgenannten Fraktionen bestätigt. Zusätzlich zeigt das erhaltene AT die vollen protease-inhibitorischen Eigenschaften.

Zusammenfassend ist also festzustellen, dass beide aktiven AT-lsoformen antiproliferative Eigenschaften bei Inkubation mit endothelialen Zellen zeigen. Die β-Isoform zeigte höhere inhibitorische Stärke als die α-lsoform. Ein AT-Konzentrat, das eine Mischung von beiden aktiven Isoformen enthält, zeigte ebenfalls inhibitorische Wirksamkeit. Die Gegenwart einer Menge (10%) von latentem AT verminderte die inhibitorische Stärke des Konzentrats nicht.

Deshalb ist ein weiterer Gegenstand der Erfindung die Verwendung der α-Isoform oder der β-Isoform oder ihrer Mischung oder eines Konzentrates von Antithrombin III zur Prophylaxe und Behandlung von Erkrankungen, die durch eine pathologische Angiogenese oder Arteriogenese verursacht werden.

Es konnte auch gezeigt werden, dass die endotheliale Zellvermehrung, die entweder durch Wachstumsfaktoren wie VEGF ('vascular endothelial growth factor' oder basischen Fibroplastenwachstumsfaktor bFGF) oder Serum hervorgerufen wurde, durch aktives AT oder ein AT-Konzentrat inhibiert werden kann. Die Verwendung einer aktiven AT-Zubereitung, hergestellt durch Immunadsorption, zeigte vergleichbare Ergebnisse und bestätigte, dass die angiogene Wirksamkeit von AT vermittelt wird und nicht etwa von anderen plasmatischen Eiweißspuren. Hieraus kann geschlossen werden, dass aktives AT, und zwar entweder die aktiven α- oder β-lsoformen allein oder als Mischung, für die Prophylaxe und Behandlung von Erkrankungen benutzt werden können, die durch eine Angionese induziert oder von ihr unterstützt oder von ihr begleitet werden wie Retinopathien, Neuropathien, rheumatoide Arthritis, Psoirasis, Endometriose, und dass sie auch benutzt werden können, um die Ausbreitung von Metastasen und das Wachstum von Tumoren zu verhindern einschließlich solcher, die induziert oder unterstützt werden durch Wachstumsfaktoren wie Zytokinen. Das gleiche gilt für die Prophylaxe und Behandlung von chronischer Bronchitis und chronischen Entzündungen des Magen-Darm-Traktes und granulomatöser und anderer infektiöser Krankheiten wie Lepra. Die Gegenwart des latenten AT vermindert nicht die gefundenen anti-angiogenen Eigenschaften, so dass eine Mischung mit aktiven α- und/oder β-AT ebenfalls benutzt werden kann. Außer aus Plasma gewonnenem Antithrombin kann auch rekombinant oder transgen hergestelltes aktives Antithrombin benutzt werden, und zwar entweder allein oder in Kombination mit latentem Antithrombin.

Antithrombin kann intravenös, subkutan, intramuskulär oder topisch (z. B. in Form von Tropfen, Salben oder als Bestandteil eines Wundverschlussmittels wie einem Vlies) eingesetzt werden. Die folgenden Beispiele zeigen die inhibitorischen Effekte, die mit den gereinigten AT-lsoformen und einem AT-Konzentrat beobachtet wurden.

### Beispiel 1

### Inhibition der VEGF-induzierten HUVEC-Vermehrung durch Antithrombin

Es konnte gezeigt werden, dass VEGF₁₀₅ eine dosisabhängige Vermehrung der HUVEC Zellzahl induzieren kann, was durch Färbung mit Kristallviolett gemessen wurde. Ein Inkubation über 48 Stunden mit 15,6 ng/ml VEGF (eine Konzentration, die eine untermaximale Wirkung erzeugt), wurde in der Gegenwart von verschiedenen Konzentrationen von unterschiedlichen Zubereitungen und Fraktionen von Antithrombin in RPME 1640 durchgeführt.

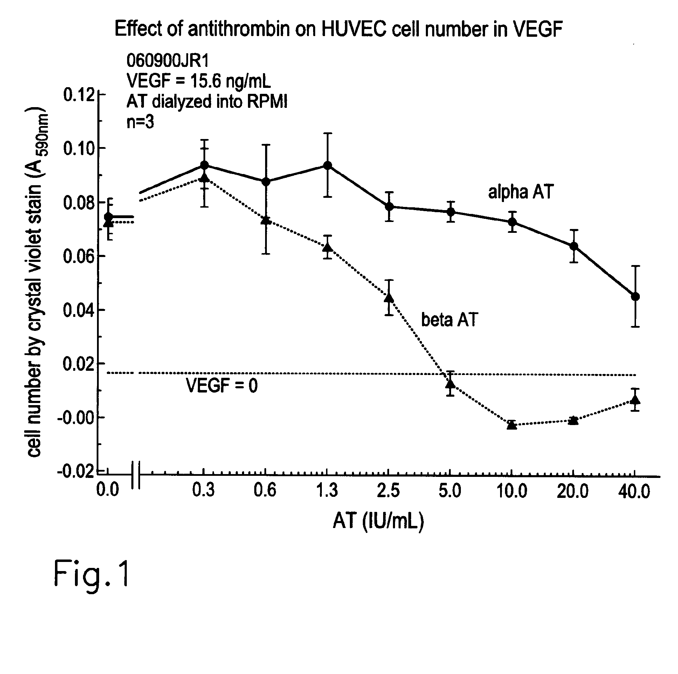

AT bewirkte eine dosisabhängige Inhibition der von VEGF induzierten Vermehrung der HUVEC-Zahl. Die β-lsoform war wirksamer als das AT-α, wie es die Fig. 1 zeigt.

### Beispiel 2

### Inhibition der endothelialen Zellvermehrung durch ein AT-Konzentrat

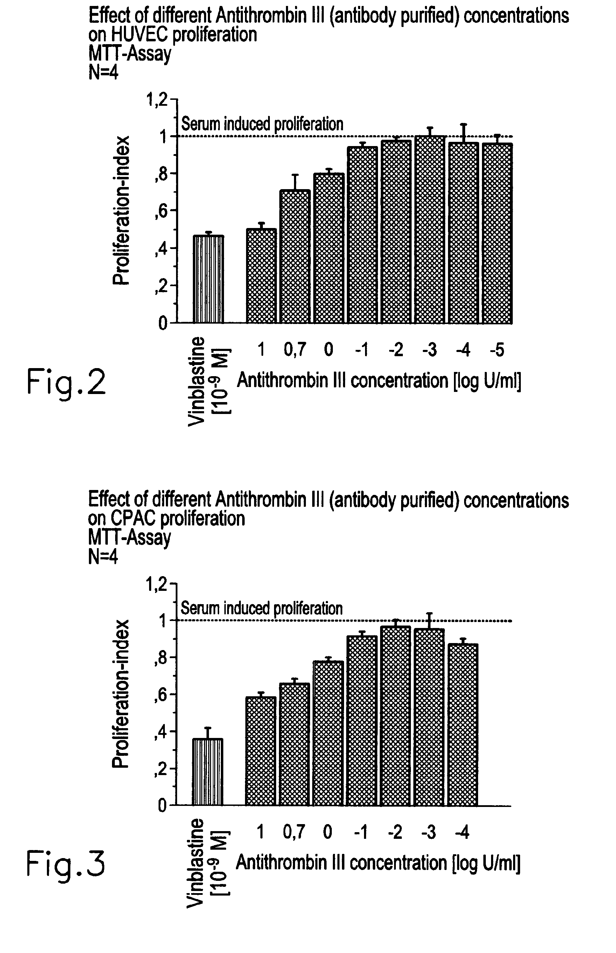

HUVEC wurde aus frischen Plazenta-Nabelschnüren isoliert und bis zur Konfluenz in einer feuchten Atmosphäre bei 37°C und 5% CO₂ wachsen gelassen. Das Wachstumsmedium war ECGM (PromoCell, Heidelberg, Deutschland) ergänzt mit 10% fötalem Kälberserum (FCS) (PAA Laboratories, Linz, Österreich). Dann wurden die Zellen voneinander durch eine Behandlung mit Kollagenase getrennt und in eine mit 96 Vertiefungen ausgestattete Gewebekulturplatte in einer Konzentration von 5 x 10³ Zellen pro Vertiefung in ein Kulturmedium, das 20% FCS enthielt, ausgesät. Nach 24 Stunden wurden die Zellen zweimal mit RPME 1640 (Biological Industries, Kibbutz Beit Haemek, Israel) gewaschen und mit den Testsubstanzen in einem Medium, das 2% FCS enthielt, 72 Stunden lang inkubiert. Als positive Kontrolle wurde Vinblastin in einer Konzentration von 10⁻⁹ M eingesetzt (siehe Fig. 2). Die antiproliferative Wirkung dieser Substanz auf HUVEC ist schon beschrieben worden (6). Eine zweite endotheliale Zelllinie, die endotheliale Zelllinie der Rinderlungenarterie CPA (ATCC, Rockville, MD) wurde zusammen mit einem Kulturmedium, das aus Earle's Medium 199 bestand (PAA Laboratories, Linz, Österreich), verwendet. Die FCS-Mengen waren wie oben beschrieben (siehe Fig. 3).

Nach Inkubation bei 37°C während der angegebenen Zeit wurde die Zellvermehrung unter Verwendung eines kolorimetrischen Testsystems gemessen. Dieses Testsystem basiert auf der Reaktion von Tetrazoliumsalz MTT (Sigma Chemical Company) in violettes Formazan durch aktive mitochondrische Dehydrogenase. Deshalb zeigt diese Reaktion lebende, aber keine toten Zellen an und das erzeugte Signal ist direkt proportional zur Anzahl der Zellen. Die MTT-Lösung wurde in einer Konzentration von 5 mg MTT/ml PBS in alle Vertiefungen der Testkulturplatte gegeben und weitere 6 Stunden inkubiert. Dann wurde DMSO (Merck) zu jeder Vertiefung hinzugefügt und die Platten weitere 30 Minuten inkubiert. Anschließend wurde die optische Dichte mit einem enzymverbundenen Immunosorbent Assay (ELISA) Reader bei 570 nm gemessen.

Um diese Ergebnisse zu bestätigen, wurde ein BrdU Testsystem (Boehringer Mannheim, Deutschland) verwendet und gemäß den Herstellervorschriften eingesetzt. Dieses Testsystem basiert auf der Messung der BrdU-Einbaus während der DNA-Synthese in sich vermehrenden Zellen.

Die Daten werden als Proliferationsindex angegeben, der das Verhältnis zwischen der seruminduzierten Zellvermehrung und der Zellvermehrung in der Gegenwart von Testsubstanzen angibt.

### Beispiel 3

### Wirkung eines AT-Konzentrats auf die Vermehrung von HUVEC und CPA

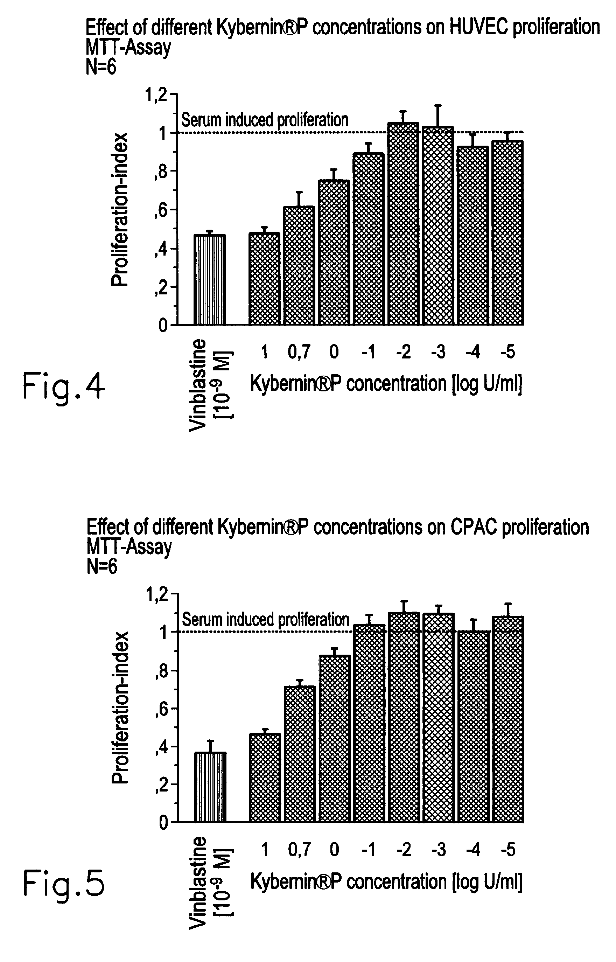

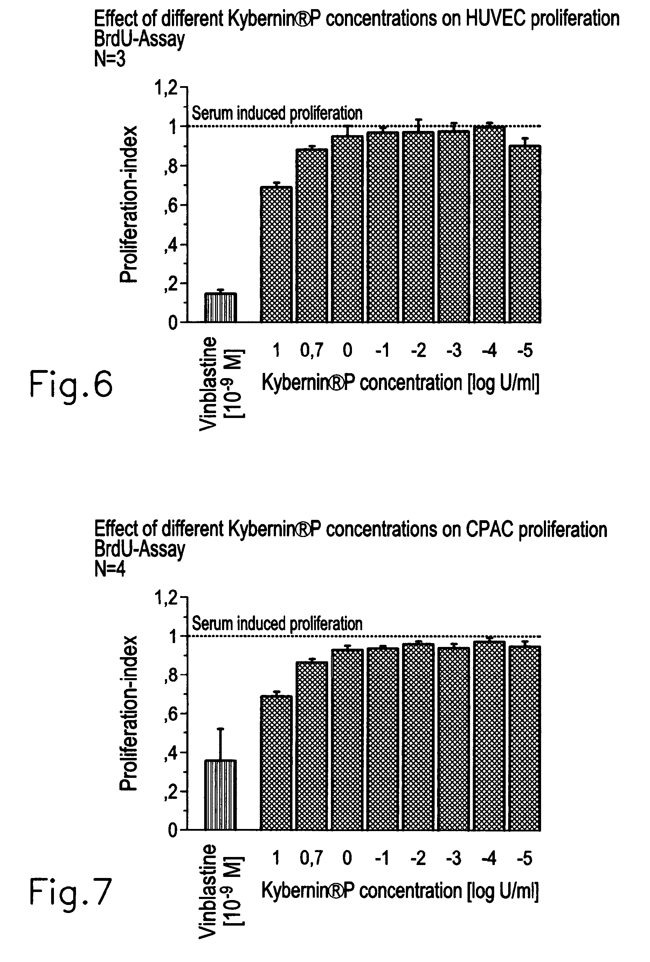

Ein AT-Konzentrat (Kybernin ®P, Aventis Behring GmbH, Deutschland), das etwa 10% latentes AT enthielt, inhibierte die Zellvermehrung von HUVEC oder CPA in einer konzentrationsabhängigen Weise (über 1 IU/ml), wenn es zum Kulturmedium vor dem Beginn der 72-stündigen Inkubation gegeben wurde. Diese Beobachtung zeigt, dass die Mischung von aktivem (bezüglich der Proteaseinhibierung und der Bindung an Heparin) und latentem AT ebenfalls inhibitorische Eigenschaften auf die Zellvermehrung zeigt. Um zu bestätigen, dass die reduzierte Zahl von endothelialen Zellen im MTT-assay (Fig. 4 und Fig. 5) tatsächlich auf die Inhibierung der DNA-Vermehrung zurückzuführen ist, wurde die Synthese in endothelialen Zellen mittels eines BrdU Einbau-assays (Fig. 6 und Fig. 7) durchgeführt. Die Ergebnisse der AT III Inhibierung auf die DNA-Synthese bei solchen Konzentraten zeigen deren antiproliferative Wirkungen.

Ein Gemisch aus gereinigten AT α und β (ohne latentes AT) zeigte ebenfalls inhibitorische Wirkung in diesen Testsystemen.
1. Carmeliet P. Mechanisms of angiogenesis and arteriogenesis. Nature Med. 2000; 6: 389-395.
2. Abdulkadir SA. Carvalhal GF. Kaleem Z., Kisiel W. Humphrey PA, Catalona WJ, Milbrandt J. Tissue factor expression and angiogenesis in human prostate carcinoma. Hum Pathol. 2000; 31 (4): 443-447.
3. Witmer MR and Hatton MW. Antithrombin III beta associates more readily than antithrombin III-alpha with uninjured and de-endothelialized aortic wall in vitro and in vivo. Arterioscler Thromb. 1991; 11 (3): 530-539.
4. Römisch J., Dönges R., Stauss H., Inthorn D., Mühlbayer D., Hoffmann JN. AT III isoform proportion in plasmas of healthy subjects, septic patients and AT III concentrates a novel method of quantitation. Intens Care Med 2000: 26 (3):S 303, A 345.
5. O'Reilly MS, Pirie-Shepherd S., Lane WS and Folkman J. Antiangiogenic activity of the cleaved conformation of the serpin antithrombin. Science. 1999; 289 (5435): 1926-1928.
6. Vacca A., lurlaro M., Ribatti D., Minischetti M., Nico B., Ria R., Pellegrino A., Dammacco F., Antiangiogenesis is produced by nontoxic doses of vinblastine. Blood. 1999: 94 (12): 4143-55.

## Patentansprüche

1. Verwendung des aktiven Antithrombin III, das Thrombin inhibitorische Eigenschaften und Affinität zu Heparin hat, für die Prophylaxe und Behandlung von Erkrankungen, die durch Angiogenese oder Arteriogenese erzeugt werden.

2. Verwendung von Antithrombin III gemäß Anspruch 1, das aktives Antithrombin enthält, zur Prophylaxe und zur Behandlung von Erkrankungen, die durch eine Angiogenese oder Arteriogenese hervorgerufen werden.

3. Verwendung von Antithrombin III gemäß den Ansprüchen 1 und 2, **dadurch gekennzeichnet, dass** die α-lsoform, die β-lsoform, Mischungen von beiden oder ein Konzentrat von Antithrombin III zur Prophylaxe und zur Behandlung von Erkrankungen benutzt werden, die durch Angiogenese oder Arteriogenese erzeugt werden.

4. Verwendung von Antithrombin III gemäß den Ansprüchen 1 bis 3, **dadurch gekennzeichnet, dass** es für die Prophylaxe und die Behandlung von Retinopathien, Neuropathien oder Infektionskrankheiten wie Lepra eingesetzt wird.

5. Verwendung von Antithrombin nach den Ansprüchen 1 bis 3, **dadurch gekennzeichnet, dass** es für die Prophylaxe und Behandlung von Krebsgeschwüren und Metastasen von Krebsgeschwüren eingesetzt wird.

6. Verwendung von Antithrombin nach den Ansprüchen 1 bis 5, **dadurch gekennzeichnet, dass** es intravenös, subkutan, intramuskulär oder topisch angewendet wird.
